# EUROPÄISCHE PATENTSCHRIFT

(11) **EP 3 725 230 B1**
(45) Veröffentlichungstag und Bekanntmachung des Hinweises auf die Patenterteilung: **25.10.2023**
(21) Anmeldenummer: 19197797.4
(22) Anmeldetag: 17.09.2019
(51) Int. Cl.: A61B 6/03, A61B 6/06, A61B 6/00, A61B 6/04

(54) **VERFAHREN ZUM ERFASSEN VON PROJEKTIONSDATEN MITTELS EINES COMPUTERTOMOGRAPHIEGERÄTS UND COMPUTERTOMOGRAPHIEGERÄT**
METHOD FOR DETECTING PROJECTION DATA BY MEANS OF A COMPUTER TOMOGRAPHY DEVICE AND COMPUTER TOMOGRAPHY DEVICE
PROCÉDÉ D'ENREGISTREMENT DES DONNÉES DE PROJECTION AU MOYEN D'UN APPAREIL DE TOMODENSITOMÉTRIE ET APPAREIL DE TOMODENSITOMÉTRIE

(43) Veröffentlichungstag der Anmeldung: 21.10.2020
(73) Patentinhaber: Siemens Healthcare GmbH, 91052 Erlangen (DE)
(72) Erfinder: Berner, Matthias, 91074 Herzogenaurach (DE); Bianconi, Maurizio, 81375 München (DE); Kühn, Ulrich, 91083 Baiersdorf (DE)

(56) Entgegenhaltungen:
- US-A1- 2012 128 120
- US-A1- 2013 308 747
- XIA YAN ET AL: "Dose reduction achieved by dynamically collimating the redundant rays in fan-beam and cone-beam CT", 2013 IEEE NUCLEAR SCIENCE SYMPOSIUM AND MEDICAL IMAGING CONFERENCE (2013 NSS/MIC), IEEE, 27. Oktober 2013 (2013-10-27), Seiten 1-4, XP032601595, DOI: 10.1109/NSSMIC.2013.6829341 [gefunden am 2014-06-10]

## Beschreibung

Die Erfindung betrifft ein Verfahren zum Erfassen von Projektionsdaten mittels eines Computertomographiegeräts. Die Erfindung betrifft ferner ein Computertomographiegerät.

US 2012/128120 A1 offenbart ein Röntgen-Computertomographie-System, aufweisend eine Röntgenquelle, einen einstellbaren Kollimator, der mit der Röntgenquelle verbunden ist und der dazu eingerichtet ist, einen Fokus des durch die Röntgenquelle erzeugten Röntgenstrahlbündels einzustellen, und ferner aufweisend eine Steuereinrichtung, die dazu eingerichtet ist, den Kollimator zu steuern, um den Fokus mit Blick auf einem interessierenden Bereich (ROI) einzustellen, und um eine Strahlintensität für das Röntgenstrahlbündel zu steuern, das durch die Röntgenquelle während eines Scandurchlaufs erzeugt wird.

US 2013/308747 A1 offenbart eine Computertomographie-Bildgebungsmodalität, umfassend eine Strahlungsquelle, die konfiguriert ist, Strahlung zu emittieren, eine Detektoranordnung, die konfiguriert ist, Strahlung zu erfassen, und einen Pre-Objekt-Kollimator, der konfiguriert ist, eine Dämpfung der emittierten Strahlung während einer Untersuchung eines Objekts in einer Fächerwinkelrichtung dynamisch einzustellen.

XIA YAN ET AL: "Dose reduction achieved by dynamically collimating the redundant rays in fan-beam and cone-beam CT", 2013 IEEE NUCLEAR SCIENCE SYMPOSIUM ANO MEDICAL IMAGING CONFERENCE (2013 NSS/MIC), IEEE, 27. Oktober 2013, offenbart einen algorithmischen Ansatz für eine Computertomographie-Bildgebung, der dynamische Kollimation verwendet, um redundante Strahlen abzuschirmen.

In der Computertomographie kann insbesondere bei einem kurzen Spiralcan mit hoher Z-Abdeckung mittels einer dynamischen Kollimierung viel Dosis eingespart werden. Dabei wird am Anfang und/oder am Ende des Spiralcans mittels einer Blende eine Lage einer Begrenzungsfläche eines Fächerstrahls an eine Lage einer Begrenzungsfläche eines abzubildenden Bereichs des Patienten dynamisch angepasst, um zu vermeiden, dass der Fächerstrahl den Patienten außerhalb des abzubildenden Bereichs durchdringt.

Insbesondere bei einem hohen Liegenpitch ist dafür eine hohe dynamische Performance erforderlich. Andererseits ist für die Schichtkollimation des Fächerstrahls eine hohe Positioniergenauigkeit erforderlich. Ein herkömmliches Computertomographiegerät weist daher zusätzlich zu dem herkömmlichen Kollimator, der für die Schichtkollimation des Fächerstrahls vorgesehen ist und nicht für hohe Geschwindigkeiten, die für die dynamische Kollimierung erforderlich sind, ausgelegt ist, ein weiteres Blendensystem auf, die speziell für die dynamische Kollimierung ausgebildet ist. Dieses weitere Blendensystem wird auch als Shuttereinheit bezeichnet.

Mit dem herkömmlichen Kollimator und der Shuttereinheit wurden somit zwei separate Blendensysteme nebeneinander verwendet, wodurch die Varianz, die Komplexität und die Kosten erhöht sind.

Die Erfindung hat die Aufgabe, eine Alternative zur herkömmlichen Kollimierung eines Fächerstrahls bereitzustellen, die in Bezug auf die Komplexität des mechanischen Aufbaus und die Positioniergenauigkeit verbessert ist. Jeder Gegenstand eines unabhängigen Anspruchs löst diese Aufgabe. In den abhängigen Ansprüchen sind weitere vorteilhafte Aspekte der Erfindung berücksichtigt.

Die Erfindung betrifft ein Verfahren zum Erfassen von Projektionsdaten mittels eines Computertomographiegeräts, das eine Strahlungsquelle zur Erzeugung einer Strahlung, einen Detektor und einen Kollimator mit einer ersten Blendenbacke und einer zweiten Blendenbacke aufweist, das Verfahren umfassend:
- Positionieren der ersten Blendenbacke und Positionieren der zweiten Blendenbacke derart, dass mittels der ersten Blendenbacke und der zweiten Blendenbacke eine Schichtkollimation eines Fächerstrahls der Strahlung eingestellt wird,
- Erfassen der Projektionsdaten eines abzubildenden Bereichs eines Patienten mittels des Detektors, während der Detektor und die Strahlungsquelle in einer Rotationsebene um eine Rotationsachse gedreht werden und gleichzeitig der abzubildende Bereich des Patienten relativ zu der Rotationsebene bewegt wird, wobei der Fächerstrahl den abzubildenden Bereich des Patienten durchdringt und auf den Detektor trifft,
- Bewegen der ersten Blendenbacke während des Erfassens der Projektionsdaten derart, dass eine Lage einer Begrenzungsfläche des Fächerstrahls an eine Lage einer Begrenzungsfläche des abzubildenden Bereichs des Patienten dynamisch angepasst wird, sodass im Wesentlichen vermieden wird, dass der Fächerstrahl den Patienten außerhalb des abzubildenden Bereichs durchdringt,
- dadurch gekennzeichnet, dass das Positionieren der ersten Blendenbacke erfolgt, indem mittels eines ersten Gewindetriebs eine erste Drehbewegung einer ersten Gewindespindel in eine erste Translationsbewegung der ersten Blendenbacke umgesetzt wird, und dass das Bewegen der ersten Blendenbacke erfolgt, indem mittels des ersten Gewindetriebs eine zweite Drehbewegung der ersten Gewindespindel in eine zweite Translationsbewegung der ersten Blendenbacke umgesetzt wird,
- wobei die erste Drehbewegung der ersten Gewindespindel und die zweite Drehbewegung der ersten Gewindespindel mittels eines ersten Schrittmotors angetrieben werden,
- wobei die zweite Drehbewegung der ersten Gewindespindel feldorientiert geregelt wird.

Auf diese Weise können die Schichtkollimation und die dynamische Kollimierung mittels derselben Blendenbacken erfolgen. Auf die zusätzliche Shuttereinheit kann somit verzichtet werden, wodurch die Varianz, die Komplexität sowie die Kosten für Hardware und Montage verringert werden können.

Eine Ausführungsform sieht vor, dass bei dem Erfassen der Projektionsdaten die Schichtkollimation des Fächerstrahls der Strahlung während mindestens einer halben Umdrehung, beispielsweise während mindestens einer ganzen Umdrehung, des Detektors und der Strahlungsquelle um die Rotationsachse konstant gehalten wird. Insbesondere kann vorgesehen sein, dass während der mindestens einen halben Umdrehung, beispielsweise während der mindestens einen ganzen Umdrehung, die Position der ersten Blendenbacke konstant gehalten wird und/oder die Position der zweiten Blendenbacke konstant gehalten wird.

Die Projektionsdaten können beispielsweise Projektionsdaten für eine Mehrschicht-Computertomographie-Bildgebungsuntersuchung sein, die dem Fachmann auch als Mehrzeilen-Computertomographie-Bildgebungsuntersuchung oder Multislice-Computertomographie-Bildgebungsuntersuchung bekannt ist.

Die Schichtkollimation des Fächerstrahls der Strahlung kann beispielsweise eingestellt werden, indem Anteile der Strahlung, die auf Bereiche des Detektors, insbesondere Zeilen des Detektors, ausgerichtet sind, die nicht für die Mehrschicht-Computertomographie-Bildgebungsuntersuchung ausgewählt sind, mittels der ersten Blendenbacke und der zweiten Blendenbacke ausgeblendet werden.

Eine Ausführungsform sieht vor, dass die Lage der Begrenzungsfläche des Fächerstrahls dynamisch an die Lage der Begrenzungsfläche des abzubildenden Bereichs des Patienten angepasst wird, bis eine vollständige Ausblendung des Fächerstrahls durch die erste Blendenbacke und die zweite Blendenbacke eintritt.

Eine Ausführungsform sieht vor, dass das Bewegen der ersten Blendenbacke und/oder das Bewegen der zweiten Blendenbacke jeweils mit einer Geschwindigkeit, beispielsweise einer Geschwindigkeit relativ und/oder senkrecht zu der Rotationsebene, von mindestens 150 Millimetern pro Sekunde, insbesondere mindestens 175 Millimetern pro Sekunde, beispielsweise mindestens 200 Millimetern pro Sekunde, zu bewegen, erfolgt, insbesondere um die Lage der Begrenzungsfläche des Fächerstrahls an die Lage der Begrenzungsfläche des abzubildenden Bereichs dynamisch anzupassen.

Eine Ausführungsform sieht vor, dass das Bewegen der ersten Blendenbacke und/oder das Bewegen der zweiten Blendenbacke jeweils eine Beschleunigung, beispielsweise eine Beschleunigung relativ und/oder senkrecht zu der Rotationsebene, von mindestens 3000 Millimetern pro Quadratsekunde, insbesondere mindestens 3500 Millimetern pro Quadratsekunde, beispielsweise mindestens 4000 Millimetern pro Quadratsekunde, umfasst, insbesondere um die Lage der Begrenzungsfläche des Fächerstrahls an die Lage der Begrenzungsfläche des abzubildenden Bereichs dynamisch anzupassen.

Das Positionieren der zweiten Blendenbacke kann beispielsweise erfolgen, indem mittels eines zweiten Gewindetriebs eine erste Drehbewegung einer zweiten Gewindespindel in eine erste Translationsbewegung der zweiten Blendenbacke umgesetzt wird. Das Bewegen der zweiten Blendenbacke kann beispielsweise erfolgen, indem mittels des zweiten Gewindetriebs eine zweite Drehbewegung der zweiten Gewindespindel in eine zweite Translationsbewegung der zweiten Blendenbacke umgesetzt wird, insbesondere um die Lage der Begrenzungsfläche des Fächerstrahls an die Lage der Begrenzungsfläche des abzubildenden Bereichs dynamisch anzupassen.

Die erste Translationsbewegung der ersten Blendenbacke und die zweite Translationsbewegung der ersten Blendenbacke können beispielsweise jeweils parallel zu der Rotationsachse erfolgen. Die erste Translationsbewegung der zweiten Blendenbacke und die zweite Translationsbewegung der zweiten Blendenbacke können beispielsweise jeweils parallel zu der Rotationsachse erfolgen.

Eine Ausführungsform sieht vor, dass die erste Drehbewegung der zweiten Gewindespindel und die zweite Drehbewegung der zweiten Gewindespindel mittels eines zweiten Schrittmotors angetrieben werden.

Eine Ausführungsform sieht vor, dass die zweite Drehbewegung der zweiten Gewindespindel feldorientiert geregelt wird. Eine Ausführungsform sieht vor, dass die erste Drehbewegung der ersten Gewindespindel feldorientiert geregelt wird und/oder dass die erste Drehbewegung der zweiten Gewindespindel feldorientiert geregelt wird.

Die Erfindung betrifft ferner ein Computertomographiegerät, aufweisend:
- eine Strahlungsquelle zur Erzeugung einer Strahlung,
- einen Detektor und
- einen Kollimator mit einer ersten Blendenbacke und einer zweiten Blendenbacke,
- wobei der Kollimator ausgebildet ist zum Positionieren der ersten Blendenbacke und zum Positionieren der zweiten Blendenbacke derart, dass mittels der ersten Blendenbacke und der zweiten Blendenbacke eine Schichtkollimation eines Fächerstrahls der Strahlung eingestellt werden kann,
- wobei der Detektor ausgebildet ist zum Erfassen der Projektionsdaten eines abzubildenden Bereichs eines Patienten, während der Detektor und die Strahlungsquelle in einer Rotationsebene um eine Rotationsachse gedreht werden und gleichzeitig der abzubildende Bereich des Patienten relativ zu der Rotationsebene bewegt wird, wobei der Fächerstrahl den abzubildenden Bereich des Patienten durchdringt und auf den Detektor trifft,
- wobei der Kollimator ferner ausgebildet ist zum Bewegen der ersten Blendenbacke während des Erfassens der Projektionsdaten derart, dass eine Lage einer Begrenzungsfläche des Fächerstrahls an eine Lage einer Begrenzungsfläche des abzubildenden Bereichs des Patienten dynamisch angepasst werden kann, sodass im Wesentlichen vermieden wird, dass der Fächerstrahl den Patienten außerhalb des abzubildenden Bereichs durchdringt,
- dadurch gekennzeichnet, dass der Kollimator eine erste Gewindespindel und einen ersten Gewindetrieb aufweist, wobei der erste Gewindetrieb dazu ausgebildet ist, eine erste Drehbewegung der ersten Gewindespindel in eine erste Translationsbewegung der ersten Blendenbacke umzusetzen, durch die das Positionieren der ersten Blendenbacke erfolgt, und eine zweite Drehbewegung der ersten Gewindespindel in eine zweite Translationsbewegung der ersten Blendenbacke umzusetzen, durch die das Bewegen der ersten Blendenbacke erfolgt,
- wobei der Kollimator einen ersten Schrittmotor zum Antreiben der ersten Drehbewegung der ersten Gewindespindel und der zweiten Drehbewegung der ersten Gewindespindel aufweist,
- wobei das Computertomographiegerät ferner eine Regelungseinheit, die zur feldorientierten Regelung der zweiten Drehbewegung der ersten Gewindespindel ausgebildet ist, aufweist.

Eine Ausführungsform sieht vor, dass der Kollimator dazu ausgebildet ist, bei dem Erfassen der Projektionsdaten die Schichtkollimation des Fächerstrahls der Strahlung während mindestens einer halben Umdrehung, beispielsweise während mindestens einer ganzen Umdrehung, des Detektors und der Strahlungsquelle um die Rotationsachse konstant zu halten. Insbesondere kann vorgesehen sein, dass der Kollimator dazu ausgebildet ist, während der mindestens einen halben Umdrehung, beispielsweise während der mindestens einen ganzen Umdrehung, die Position der ersten Blendenbacke konstant zu halten und/oder die Position der zweiten Blendenbacke konstant zu halten.

Eine Ausführungsform sieht vor, dass der Kollimator dazu ausgebildet ist, die Lage der Begrenzungsfläche des Fächerstrahls dynamisch an die Lage der Begrenzungsfläche des abzubildenden Bereichs des Patienten anzupassen, bis eine vollständige Ausblendung des Fächerstrahls durch die erste Blendenbacke und die zweite Blendenbacke eintritt.

Eine Ausführungsform sieht vor, dass der Kollimator dazu ausgebildet ist, die erste Blendenbacke und/oder die zweite Blendenbacke jeweils mit einer Geschwindigkeit, beispielsweise einer Geschwindigkeit relativ und/oder senkrecht zu der Rotationsebene, von mindestens 150 Millimetern pro Sekunde, insbesondere mindestens 175 Millimetern pro Sekunde, beispielsweise mindestens 200 Millimetern pro Sekunde, zu bewegen, insbesondere um die Lage der Begrenzungsfläche des Fächerstrahls an die Lage der Begrenzungsfläche des abzubildenden Bereichs dynamisch anzupassen.

Eine Ausführungsform sieht vor, dass der Kollimator dazu ausgebildet ist, die erste Blendenbacke und/oder die zweite Blendenbacke jeweils mit einer Beschleunigung, beispielsweise einer Beschleunigung relativ und/oder senkrecht zu der Rotationsebene, von mindestens 3000 Millimetern pro Quadratsekunde, insbesondere mindestens 3500 Millimetern pro Quadratsekunde, beispielsweise mindestens 4000 Millimetern pro Quadratsekunde, zu bewegen, insbesondere um die Lage der Begrenzungsfläche des Fächerstrahls an die Lage der Begrenzungsfläche des abzubildenden Bereichs dynamisch anzupassen.

Eine Ausführungsform sieht vor, dass der Kollimator eine zweite Gewindespindel und einen zweiten Gewindetrieb aufweist, wobei der zweite Gewindetrieb dazu ausgebildet ist, eine erste Drehbewegung der zweiten Gewindespindel in eine erste Translationsbewegung der zweiten Blendenbacke umzusetzen, durch die das Positionieren der zweiten Blendenbacke erfolgt, und/oder eine zweite Drehbewegung der zweiten Gewindespindel in eine zweite Translationsbewegung der zweiten Blendenbacke umzusetzen, durch die das Bewegen der zweiten Blendenbacke erfolgt.

Eine Ausführungsform sieht vor, dass eine Gewindesteigung der ersten Gewindespindel mindestens 3 Millimeter pro Umdrehung, insbesondere mindestens 3,5 Millimeter pro Umdrehung, beispielsweise mindestens 4 Millimeter pro Umdrehung, beträgt und/oder dass eine Gewindesteigung der zweiten Gewindespindel mindestens 3 Millimeter pro Umdrehung, insbesondere mindestens 3,5 Millimeter pro Umdrehung, beispielsweise mindestens 4 Millimeter pro Umdrehung, beträgt.

Eine Ausführungsform sieht vor, dass der Kollimator einen zweiten Schrittmotor zum Antreiben der ersten Drehbewegung der zweiten Gewindespindel und der zweiten Drehbewegung der zweiten Gewindespindel aufweist.

Eine Ausführungsform sieht vor, dass das Computertomographiegerät ferner eine Regelungseinheit aufweist, die zur feldorientierten Regelung der zweiten Drehbewegung der zweiten Gewindespindel ausgebildet ist.

Eine Ausführungsform sieht vor, dass die Regelungseinheit zur feldorientierten Regelung der ersten Drehbewegung der ersten Gewindespindel und/oder zur feldorientierten Regelung der ersten Drehbewegung der zweiten Gewindespindel ausgebildet ist.

Die Regelungseinheit kann beispielsweise mindestens ein Field Programmable Gate Array (FPGA) aufweisen. Die feldorientierte Regelung kann insbesondere in dem Field Programmable Gate Array (FPGA) implementiert sein.

Dadurch ist es möglich, einerseits eine hohe Geschwindigkeit für das Bewegen der Blendenbacken und andererseits eine hohe Positioniergenauigkeit für das Positionieren der Blendenbacken zu erreichen.

Im Rahmen der Erfindung können Merkmale, welche in Bezug auf unterschiedliche Ausführungsformen der Erfindung und/oder unterschiedliche Anspruchskategorien (Verfahren, Verwendung, Vorrichtung, System, Anordnung usw.) beschrieben sind, zu weiteren Ausführungsformen der Erfindung kombiniert werden. Beispielsweise kann ein Anspruch, der eine Vorrichtung betrifft, auch mit Merkmalen, die im Zusammenhang mit einem Verfahren beschrieben oder beansprucht sind, weitergebildet werden und umgekehrt. Funktionale Merkmale eines Verfahrens können dabei durch entsprechend ausgebildete gegenständliche Komponenten ausgeführt werden. Neben den in dieser Anmeldung ausdrücklich beschriebenen Ausführungsformen der Erfindung sind vielfältige weitere Ausführungsformen der Erfindung denkbar, zu denen der Fachmann gelangen kann, ohne den Bereich der Erfindung zu verlassen, der durch die Ansprüche vorgegeben ist.

Die Verwendung der unbestimmten Artikel "ein" bzw. "eine" schließt nicht aus, dass das betroffene Merkmal auch mehrfach vorhanden sein kann. Die Verwendung des Ausdrucks "aufweisen" schließt nicht aus, dass die mittels des Ausdrucks "aufweisen" verknüpften Begriffe identisch sein können. Beispielsweise weist das Computertomographiegerät das Computertomographiegerät auf. Die Verwendung des Ausdrucks "Einheit" schließt nicht aus, dass der Gegenstand, auf den sich der Ausdruck "Einheit" bezieht, mehrere Komponenten aufweisen kann, die räumlich voneinander separiert sind.

Im Folgenden wird die Erfindung anhand von Ausführungsbeispielen unter Hinweis auf die beigefügten Figuren erläutert. Die Darstellung in den Figuren ist schematisch, stark vereinfacht und nicht zwingend maßstabsgetreu.

Es zeigen:
die Fig. 1 einen Kollimator,
die Fig. 2 eine Darstellung einer Mehrschicht-Computertomographie-Bildgebungsuntersuchung eines Patienten unter Verwendung des Kollimators,
die Fig. 3 ein Ablaufdiagramm eines Verfahrens zum Erfassen von Projektionsdaten mittels eines Computertomographiegeräts, und
die Fig. 4 ein Computertomographiegerät.

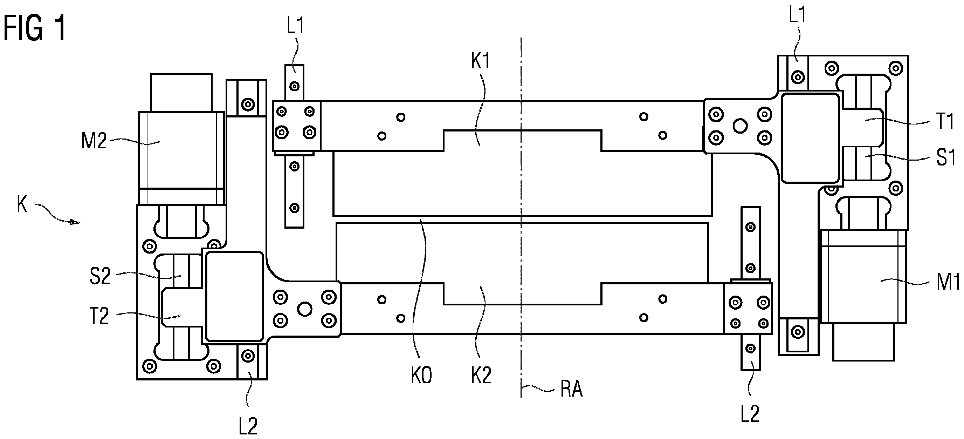

Die Fig. 1 zeigt den Kollimator K mit der ersten Blendenbacke K1 und der zweiten Blendenbacke K2. Die erste Blendenbacke ist in der ersten Schienenführung L1 gelagert. Die zweite Blendenbacke ist in der zweiten Schienenführung L2 gelagert. Die erste Blendenbacke K1 und die zweite Blendenbacke K2 sind jeweils gebogen ausgebildet und zueinander koaxial angeordnet. Die zweite Blendenbacke K2 hat einen geringeren Krümmungsradius als die erste Blendenbacke K1, so dass die erste Blendenbacke K1 und die zweite Blendenbacke K2 aus Sicht der Strahlungsquelle 26 zumindest teilweise überlappend angeordnet werden können.

Der Kollimator K weist die erste Gewindespindel S1 und den ersten Gewindetrieb T1 auf, wobei der erste Gewindetrieb T1 dazu ausgebildet ist, eine erste Drehbewegung der ersten Gewindespindel S1 in eine erste Translationsbewegung der ersten Blendenbacke K1 umzusetzen, durch die das Positionieren PK1 der ersten Blendenbacke K1 erfolgt, und eine zweite Drehbewegung der ersten Gewindespindel S1 in eine zweite Translationsbewegung der ersten Blendenbacke K1 umzusetzen, durch die das Bewegen BK1 der ersten Blendenbacke K1 erfolgt.

Der Kollimator K weist die zweite Gewindespindel S2 und den zweiten Gewindetrieb T2 auf, wobei der zweite Gewindetrieb T2 dazu ausgebildet ist, eine erste Drehbewegung der zweiten Gewindespindel S2 in eine erste Translationsbewegung der zweiten Blendenbacke K2 umzusetzen, durch die das Positionieren PK2 der zweiten Blendenbacke K2 erfolgt, und eine zweite Drehbewegung der zweiten Gewindespindel S2 in eine zweite Translationsbewegung der zweiten Blendenbacke K2 umzusetzen, durch die das Bewegen BK2 der zweiten Blendenbacke K2 erfolgt.

Der Kollimator K weist den ersten Schrittmotor M1 zum Antreiben der ersten Drehbewegung der ersten Gewindespindel S1 und der zweiten Drehbewegung der ersten Gewindespindel S1 auf. Der Kollimator K weist den zweiten Schrittmotor M2 zum Antreiben der ersten Drehbewegung der zweiten Gewindespindel S2 und der zweiten Drehbewegung der zweiten Gewindespindel S2 auf.

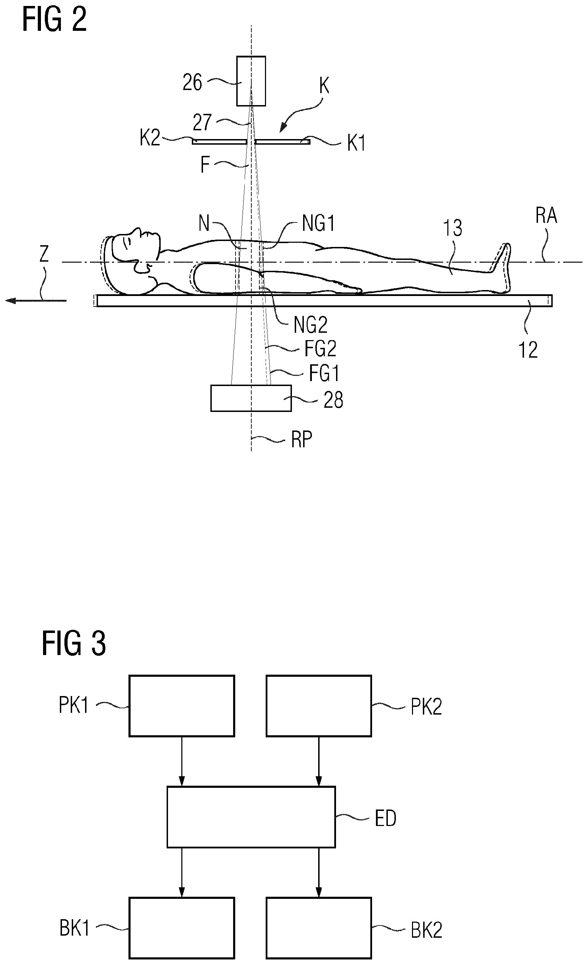

Die Fig. 2 zeigt eine Darstellung einer Mehrschicht-Computertomographie-Bildgebungsuntersuchung des Patienten 13 unter Verwendung des Kollimators K.

Der Kollimator K ist ausgebildet zum Positionieren PK1 der ersten Blendenbacke K1 und zum Positionieren PK2 der zweiten Blendenbacke K2 derart, dass mittels der ersten Blendenbacke K1 und der zweiten Blendenbacke K2 eine Schichtkollimation des Fächerstrahls F der Strahlung 27 eingestellt werden kann. Dabei dringt ein Teil der Strahlung 27 durch den Kollimatorspalt K0, der zwischen der ersten Blendenbacke K1 und der zweiten Blendenbacke K2 ausgebildet ist.

Der Detektor 28 ist ausgebildet zum Erfassen ED der Projektionsdaten eines abzubildenden Bereichs N eines Patienten 13, während der Detektor 28 und die Strahlungsquelle 26 in einer Rotationsebene RP um die Rotationsachse RA gedreht werden und gleichzeitig der abzubildende Bereich N des Patienten 13 relativ zu der Rotationsebene RP bewegt wird, wobei der Fächerstrahl F den abzubildenden Bereich N des Patienten 13 durchdringt und auf den Detektor 28 trifft.

Der Kollimator K ist ferner ausgebildet zum Bewegen BK1 der ersten Blendenbacke K1 und/oder zum Bewegen BK2 der zweiten Blendenbacke K2 während des Erfassens ED der Projektionsdaten derart, dass eine Lage einer Begrenzungsfläche des Fächerstrahls F an eine Lage einer Begrenzungsfläche des abzubildenden Bereichs N des Patienten 13 dynamisch angepasst werden kann, sodass im Wesentlichen vermieden wird, dass der Fächerstrahl F den Patienten 13 außerhalb des abzubildenden Bereichs N durchdringt.

Der Patient 13 und damit dessen abzubildender Bereich N werden mittels der Patientenlagerungsvorrichtung 10 in die Richtung Z, die parallel zu der Rotationsachse RA ist, bewegt. Aufgrund der Bewegung des abzubildenden Bereichs N des Patienten 13 relativ zu der Rotationsebene RP ändert sich die Lage der Begrenzungsfläche des abzubildenden Bereichs N des Patienten 13 von der Lage NG1 zu der Lage NG2.

Aufgrund der Bewegung der ersten Blendenbacke K1 in die Richtung Z ändert sich die Lage der Begrenzungsfläche des Fächerstrahls F von der Lage FG1 zu der Lage FG2. Dabei wird ein Anteil der Strahlung 27, der in der Lage FG1 noch Teil des Fächerstrahls F war, von der ersten Blendenbacke K1 ausgeblendet, sodass dieser Anteil der Strahlung 27 in der Lage FG2 nicht mehr Teil des Fächerstrahls F ist. Wenn dieser Anteil der Strahlung 27 nicht ausgeblendet wird, durchdringt er den Patienten 13 außerhalb des abzubildenden Bereichs N, wenn sich dessen Begrenzungsfläche in der Lage NG2 befindet.

Der Kollimator K ist dazu ausgebildet, bei dem Erfassen ED der Projektionsdaten die Schichtkollimation des Fächerstrahls F der Strahlung 27 während mindestens einer halben Umdrehung des Detektors 28 und der Strahlungsquelle 26 um die Rotationsachse RA konstant zu halten.

Der Kollimator K ist dazu ausgebildet, die Lage der Begrenzungsfläche FG des Fächerstrahls F dynamisch an die Lage der Begrenzungsfläche NG des abzubildenden Bereichs N des Patienten 13 anzupassen, bis eine vollständige Ausblendung des Fächerstrahls F durch die erste Blendenbacke K1 und die zweite Blendenbacke K2, beispielsweise aufgrund eines vollständigen Verschließens des Kollimatorspalts K0, eintritt. Das Verschließen des Kollimatorspalts K0 kann insbesondere synchron zu einem abschließenden Abschnitt des Spiralscans erfolgen.

Die Fig. 3 zeigt ein Ablaufdiagramm eines Verfahren zum Erfassen von Projektionsdaten mittels des Computertomographiegeräts 1, das die Strahlungsquelle 26 zur Erzeugung der Strahlung 27, den Detektor 28 und den Kollimator K mit der ersten Blendenbacke K1 und der zweiten Blendenbacke K2 aufweist, das Verfahren umfassend:
- Positionieren PK1 der ersten Blendenbacke K1 und Positionieren PK2 der zweiten Blendenbacke K2 derart, dass mittels der ersten Blendenbacke K1 und der zweiten Blendenbacke K2 eine Schichtkollimation des Fächerstrahls F der Strahlung 27 eingestellt wird,
- Erfassen ED der Projektionsdaten des abzubildenden Bereichs N des Patienten 13 mittels des Detektors 28, während der Detektor 28 und die Strahlungsquelle 26 in der Rotationsebene RP um die Rotationsachse RA gedreht werden und gleichzeitig der abzubildende Bereich N des Patienten 13 relativ zu der Rotationsebene RP bewegt wird, wobei der Fächerstrahl F den abzubildenden Bereich N des Patienten 13 durchdringt und auf den Detektor 28 trifft,
- Bewegen BK1 der ersten Blendenbacke K1 und/oder Bewegen BK2 der zweiten Blendenbacke K2 während des Erfassens ED der Projektionsdaten derart, dass eine Lage FG1, FG2 einer Begrenzungsfläche des Fächerstrahls F an eine Lage NG1, NG2 einer Begrenzungsfläche des abzubildenden Bereichs N des Patienten 13 dynamisch angepasst wird, sodass im Wesentlichen vermieden wird, dass der Fächerstrahl F den Patienten 13 außerhalb des abzubildenden Bereichs N durchdringt.

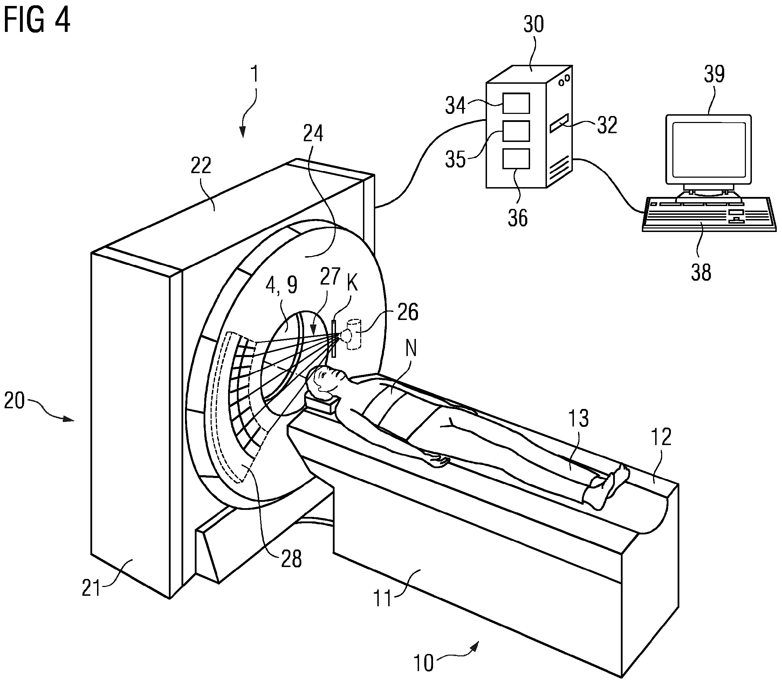

Die Fig. 4 zeigt das Computertomographiegerät 1, aufweisend die Strahlungsquelle 26 zur Erzeugung einer Strahlung 27, den Detektor 28, den Kollimator K und die Regelungseinheit 35, die zur feldorientierten Regelung der zweiten Drehbewegung der ersten Gewindespindel S1 und zur feldorientierten Regelung der zweiten Drehbewegung der zweiten Gewindespindel S2 ausgebildet ist.

Das Computertomographiegerät 1 weist ferner die Gantry 20, die tunnelförmige Öffnung 9, die Patientenlagerungsvorrichtung 10 und die Steuerungsvorrichtung 30 auf. Die Gantry 20 weist den stationären Tragrahmen 21, den Kipprahmen 22 und den Rotor 24 auf.

Der Kipprahmen 22 ist mittels einer Kipplagerungsvorrichtung an dem stationären Tragrahmen 21 um eine Kippachse relativ zu dem stationären Tragrahmen 21 kippbar angeordnet. Der Rotor 24 ist mittels einer Drehlagerungsvorrichtung an dem Kipprahmen 22 um die Rotationsachse RA relativ zu dem Kipprahmen 22 drehbar angeordnet. Die Rotationsachse RA ist senkrecht zu der Kippachse.

In die tunnelförmige Öffnung 9 ist der Patient 13 einführbar. In der tunnelförmigen Öffnung 9 befindet sich der Akquisitionsbereich 4. In dem Akquisitionsbereich 4 ist der abzubildender Bereich N des Patienten 13 derart positionierbar, dass die Strahlung 27 von der Strahlungsquelle 26 zu dem abzubildenden Bereich gelangen kann und nach einer Wechselwirkung mit dem abzubildenden Bereich zu dem Strahlungsdetektor 28 gelangen kann.

Die Patientenlagerungsvorrichtung 10 weist den Lagerungssockel 11 und die Lagerungsplatte 12 zur Lagerung des Patienten 13 auf. Die Lagerungsplatte 12 ist derart relativ zu dem Lagerungssockel 11 bewegbar an dem Lagerungssockel 11 angeordnet, dass die Lagerungsplatte 12 in einer Längsrichtung der Lagerungsplatte 12 in den Akquisitionsbereich 4 einführbar ist.

Die Steuerungsvorrichtung 30 ist zum Empfangen der von dem Detektor erfassten Projektionsdaten ausgebildet. Die Steuerungsvorrichtung 30 ist zum Steuern des Computertomographiegeräts 1 ausgebildet. Die Steuerungsvorrichtung 30 weist die Regelungseinheit 35, das computerlesbare Medium 32 und das Prozessorsystem 36 auf. Die Steuerungsvorrichtung 30, insbesondere die Regelungseinheit 35, wird von einem Datenverarbeitungssystem, welches einen Computer aufweist, gebildet.

Die Steuerungsvorrichtung 30 weist die Bildrekonstruktionseinrichtung 34 auf. Mittels der Bildrekonstruktionseinrichtung 34 kann basierend auf den Projektionsdaten ein medizinischer Bilddatensatz rekonstruiert werden.

Das Computertomographiegerät 1 weist eine Eingabevorrichtung 38 und eine Ausgabevorrichtung 39 auf, welche jeweils mit der Steuerungsvorrichtung 30 verbunden sind. Die Eingabevorrichtung 38 ist zum Eingeben von Steuerungs-Informationen, z. B. Bildrekonstruktionsparametern, Untersuchungsparametern oder ähnliches, ausgebildet. Die Ausgabevorrichtung 39 ist insbesondere zum Ausgeben von Steuerungs-Informationen, Bildern und/oder akustischen Signalen ausgebildet.

## Patentansprüche

1. Verfahren zum Erfassen von Projektionsdaten mittels eines Computertomographiegeräts (1), das eine Strahlungsquelle (26) zur Erzeugung einer Strahlung (27), einen Detektor (28) und einen Kollimator (K) mit einer ersten Blendenbacke (K1) und einer zweiten Blendenbacke (K2) aufweist, das Verfahren umfassend:
- Positionieren (PK1) der ersten Blendenbacke (K1) und Positionieren (PK2) der zweiten Blendenbacke (K2) derart, dass mittels der ersten Blendenbacke (K1) und der zweiten Blendenbacke (K2) eine Schichtkollimation eines Fächerstrahls (F) der Strahlung (27) eingestellt wird,
- Erfassen (ED) der Projektionsdaten eines abzubildenden Bereichs (N) eines Patienten (13) mittels des Detektors (28), während der Detektor (28) und die Strahlungsquelle (26) in einer Rotationsebene (RP) um eine Rotationsachse (RA) gedreht werden und gleichzeitig der abzubildende Bereich (N) des Patienten (13) relativ zu der Rotationsebene (RP) bewegt wird, wobei der Fächerstrahl (F) den abzubildenden Bereich (N) des Patienten (13) durchdringt und auf den Detektor (28) trifft,
- Bewegen (BK1) der ersten Blendenbacke (K1) während des Erfassens (ED) der Projektionsdaten derart, dass eine Lage (FG1, FG2) einer Begrenzungsfläche des Fächerstrahls (F) an eine Lage (NG1, NG2) einer Begrenzungsfläche des abzubildenden Bereichs (N) des Patienten (13) dynamisch angepasst wird, sodass im Wesentlichen vermieden wird, dass der Fächerstrahl (F) den Patienten (13) außerhalb des abzubildenden Bereichs (N) durchdringt,
- **dadurch gekennzeichnet, dass** das Positionieren (PK1) der ersten Blendenbacke (K1) erfolgt, indem mittels eines ersten Gewindetriebs (T1) eine erste Drehbewegung einer ersten Gewindespindel (S1) in eine erste Translationsbewegung der ersten Blendenbacke (K1) umgesetzt wird, und dass das Bewegen (BK1) der ersten Blendenbacke (K1) erfolgt, indem mittels des ersten Gewindetriebs (T1) eine zweite Drehbewegung der ersten Gewindespindel (S1) in eine zweite Translationsbewegung der ersten Blendenbacke (K1) umgesetzt wird,
- wobei die erste Drehbewegung der ersten Gewindespindel (S1) und die zweite Drehbewegung der ersten Gewindespindel (S1) mittels eines ersten Schrittmotors (M1) angetrieben werden,
- wobei die zweite Drehbewegung der ersten Gewindespindel (S1) feldorientiert geregelt wird.

2. Verfahren nach Anspruch 1,
- wobei bei dem Erfassen (ED) der Projektionsdaten die Schichtkollimation des Fächerstrahls (F) der Strahlung (27) während mindestens einer halben Umdrehung des Detektors (28) und der Strahlungsquelle (26) um die Rotationsachse (RA) konstant gehalten wird.

3. Verfahren nach Anspruch 1 oder 2,
- wobei die Lage der Begrenzungsfläche (FG) des Fächerstrahls (F) dynamisch an die Lage der Begrenzungsfläche (NG) des abzubildenden Bereichs (N) des Patienten (13) angepasst wird, bis eine vollständige Ausblendung des Fächerstrahls (F) durch die erste Blendenbacke (K1) und die zweite Blendenbacke (K2) eintritt.

4. Verfahren nach einem der Ansprüche 1 bis 3,
- wobei das Bewegen (BK1) der ersten Blendenbacke (K1) mit einer Geschwindigkeit von mindestens 150 Millimetern pro Sekunde erfolgt.

5. Verfahren nach einem der Ansprüche 1 bis 4,
- wobei ein Bewegen (BK2) der zweiten Blendenbacke (K2) während des Erfassens (ED) der Projektionsdaten derart erfolgt, dass die Lage (FG1, FG2) der Begrenzungsfläche des Fächerstrahls (F) an die Lage (NG1, NG2) der Begrenzungsfläche des abzubildenden Bereichs (N) des Patienten (13) dynamisch angepasst wird, sodass im Wesentlichen vermieden wird, dass der Fächerstrahl (F) den Patienten (13) außerhalb des abzubildenden Bereichs (N) durchdringt,
- wobei das Positionieren (PK2) der zweiten Blendenbacke (K2) erfolgt, indem mittels eines zweiten Gewindetriebs (T2) eine erste Drehbewegung einer zweiten Gewindespindel (S2) in eine erste Translationsbewegung der zweiten Blendenbacke (K2) umgesetzt wird, und wobei das Bewegen (BK2) der zweiten Blendenbacke (K2) erfolgt, indem mittels des zweiten Gewindetriebs (T2) eine zweite Drehbewegung der zweiten Gewindespindel (S2) in eine zweite Translationsbewegung der zweiten Blendenbacke (K2) umgesetzt wird.

6. Verfahren nach Anspruch 5,
- wobei die erste Drehbewegung der zweiten Gewindespindel (S2) und die zweite Drehbewegung der zweiten Gewindespindel (S2) mittels eines zweiten Schrittmotors (M2) angetrieben werden,
- wobei die zweite Drehbewegung der zweiten Gewindespindel (S2) feldorientiert geregelt wird.

7. Verfahren nach Anspruch 5 oder 6,
- wobei das Bewegen (BK2) der zweiten Blendenbacke (K2) mit einer Geschwindigkeit von mindestens 150 Millimetern pro Sekunde erfolgt.

8. Computertomographiegerät (1), aufweisend:
- eine Strahlungsquelle (26) zur Erzeugung einer Strahlung (27),
- einen Detektor (28) und
- einen Kollimator (K) mit einer ersten Blendenbacke (K1) und einer zweiten Blendenbacke (K2),
- wobei der Kollimator (K) ausgebildet ist zum Positionieren (PK1) der ersten Blendenbacke (K1) und zum Positionieren (PK2) der zweiten Blendenbacke (K2) derart, dass mittels der ersten Blendenbacke (K1) und der zweiten Blendenbacke (K2) eine Schichtkollimation eines Fächerstrahls (F) der Strahlung (27) eingestellt werden kann,
- wobei der Detektor (28) ausgebildet ist zum Erfassen (ED) der Projektionsdaten eines abzubildenden Bereichs (N) eines Patienten (13), während der Detektor (28) und die Strahlungsquelle (26) in einer Rotationsebene (RP) um eine Rotationsachse (RA) gedreht werden und gleichzeitig der abzubildende Bereich (N) des Patienten (13) relativ zu der Rotationsebene (RP) bewegt wird, wobei der Fächerstrahl (F) den abzubildenden Bereich (N) des Patienten (13) durchdringt und auf den Detektor (28) trifft,
- wobei der Kollimator (K) ferner ausgebildet ist zum Bewegen (BK1) der ersten Blendenbacke (K1) während des Erfassens (ED) der Projektionsdaten derart, dass eine Lage (FG1, FG2) einer Begrenzungsfläche des Fächerstrahls (F) an eine Lage (NG1, NG2) einer Begrenzungsfläche des abzubildenden Bereichs (N) des Patienten (13) dynamisch angepasst werden kann, sodass im Wesentlichen vermieden wird, dass der Fächerstrahl (F) den Patienten (13) außerhalb des abzubildenden Bereichs (N) durchdringt,
- **dadurch gekennzeichnet, dass** der Kollimator (K) eine erste Gewindespindel (S1) und einen ersten Gewindetrieb (T1) aufweist, wobei der erste Gewindetrieb (T1) dazu ausgebildet ist, eine erste Drehbewegung der ersten Gewindespindel (S1) in eine erste Translationsbewegung der ersten Blendenbacke (K1) umzusetzen, durch die das Positionieren (PK1) der ersten Blendenbacke (K1) erfolgt, und eine zweite Drehbewegung der ersten Gewindespindel (S1) in eine zweite Translationsbewegung der ersten Blendenbacke (K1) umzusetzen, durch die das Bewegen (BK1) der ersten Blendenbacke (K1) erfolgt,
- wobei der Kollimator (K) einen ersten Schrittmotor (M1) zum Antreiben der ersten Drehbewegung der ersten Gewindespindel (S1) und der zweiten Drehbewegung der ersten Gewindespindel (S1) aufweist,
- wobei das Computertomographiegerät (1) ferner eine Regelungseinheit (35), die zur feldorientierten Regelung der zweiten Drehbewegung der ersten Gewindespindel (S1) ausgebildet ist, aufweist.

9. Computertomographiegerät (1) nach Anspruch 8,
- wobei der Kollimator (K) dazu ausgebildet ist, bei dem Erfassen (ED) der Projektionsdaten die Schichtkollimation des Fächerstrahls (F) der Strahlung (27) während mindestens einer halben Umdrehung des Detektors (28) und der Strahlungsquelle (26) um die Rotationsachse (RA) konstant zu halten.

10. Computertomographiegerät (1) nach Anspruch 8 oder 9,
- wobei der Kollimator (K) dazu ausgebildet ist, die Lage der Begrenzungsfläche (FG) des Fächerstrahls (F) dynamisch an die Lage der Begrenzungsfläche (NG) des abzubildenden Bereichs (N) des Patienten (13) anzupassen, bis eine vollständige Ausblendung des Fächerstrahls (F) durch die erste Blendenbacke (K1) und die zweite Blendenbacke (K2) eintritt.

11. Computertomographiegerät (1) nach einem der Ansprüche 8 bis 10,
- wobei der Kollimator (K) dazu ausgebildet ist, die erste Blendenbacke (K1) und/oder die zweite Blendenbacke (K2) jeweils mit einer Geschwindigkeit von mindestens 150 Millimetern pro Sekunde zu bewegen.

12. Computertomographiegerät (1) nach einem der Ansprüche 8 bis 11,
- wobei der Kollimator (K) ferner ausgebildet ist zum Bewegen (BK2) der zweiten Blendenbacke (K2) während des Erfassens (ED) der Projektionsdaten derart, dass die Lage (FG1, FG2) der Begrenzungsfläche des Fächerstrahls (F) an die Lage (NG1, NG2) der Begrenzungsfläche des abzubildenden Bereichs (N) des Patienten (13) dynamisch angepasst werden kann, sodass im Wesentlichen vermieden wird, dass der Fächerstrahl (F) den Patienten (13) außerhalb des abzubildenden Bereichs (N) durchdringt,
- wobei der Kollimator (K) eine zweite Gewindespindel (S2) und einen zweiten Gewindetrieb (T2) aufweist, wobei der zweite Gewindetrieb (T2) dazu ausgebildet ist, eine erste Drehbewegung der zweiten Gewindespindel (S2) in eine erste Translationsbewegung der zweiten Blendenbacke (K2) umzusetzen, durch die das Positionieren (PK2) der zweiten Blendenbacke (K2) erfolgt, und eine zweite Drehbewegung der zweiten Gewindespindel (S2) in eine zweite Translationsbewegung der zweiten Blendenbacke (K2) umzusetzen, durch die das Bewegen (BK2) der zweiten Blendenbacke (K2) erfolgt.

13. Computertomographiegerät (1) nach Anspruch 12,
- wobei eine Gewindesteigung der zweiten Gewindespindel (S2) mindestens 3 Millimeter pro Umdrehung beträgt.

14. Computertomographiegerät (1) nach Anspruch 12 oder 13,
- wobei der Kollimator (K) einen zweiten Schrittmotor (M2) zum Antreiben der ersten Drehbewegung der zweiten Gewindespindel (S2) und der zweiten Drehbewegung der zweiten Gewindespindel (S2) aufweist,
- wobei die Regelungseinheit (35) zur feldorientierten Regelung der zweiten Drehbewegung der zweiten Gewindespindel (S2) ausgebildet ist.

15. Computertomographiegerät (1) nach einem der Ansprüche 8 bis 14,
- wobei eine Gewindesteigung der ersten Gewindespindel (S1) mindestens 3 Millimeter pro Umdrehung beträgt.

## Claims

1. Method for capturing projection data by means of a computed tomography device (1), which has a radiation source (26) for generating radiation (27), a detector (28) and a collimator (K) having a first diaphragm jaw (K1) and a second diaphragm jaw (K2), the method comprising:
- Positioning (PK1) of the first diaphragm jaw (K1) and positioning (PK2) of the second diaphragm jaw (K2) in such a manner that a layer collimation of a fan beam (F) of the radiation (27) is set by means of the first diaphragm jaw (K1) and of the second diaphragm jaw (K2),
- Capturing (ED) the projection data of a region (N) of a patient (13) to be imaged by means of the detector (28), while the detector (28) and the radiation source (26) are rotated in a plane of rotation (RP) about an axis of rotation (RA) and at the same time the region (N) of the patient (13) to be imaged is moved relative to the plane of rotation (RP), wherein the fan beam (F) penetrates the region (N) of the patient (13) to be imaged and strikes the detector (28),
- Moving (BK1) the first diaphragm jaw (K1) during the capturing (ED) of the projection data in such a way that a position (FG1, FG2) of a boundary surface of the fan beam (F) is dynamically adjusted to a position (NG1, NG2) of a boundary surface of the region (N) of the patient (13) to be imaged, such that the fan beam (F) penetrating the patient (13) outside the region to be imaged (N) is essentially avoided,
- **characterised in that** the positioning (PK1) of the first diaphragm jaw (K1) takes place **in that** a first rotational movement of a first threaded spindle (S1) is converted into a first translational movement of the first diaphragm jaw (K1) by means of a first threaded drive (T1), and **in that** the movement (BK1) of the first diaphragm jaw (K1) takes place **in that** a second rotational movement of the first threaded spindle (S1) is converted into a second translational movement of the first diaphragm jaw (K1) by means of the first threaded drive (T1),
- wherein the first rotational movement of the first threaded spindle (S1) and the second rotational movement of the first threaded spindle (S1) are driven by means of a first stepper motor (M1),
- wherein the second rotational movement of the first threaded spindle (S1) is controlled in a field-oriented manner.

2. Method according to claim 1,
- wherein during the capturing (ED) of the projection data, the layer collimation of the fan beam (F) of the radiation (27) is kept constant for at least one half revolution of the detector (28) and the radiation source (26) about the axis of rotation (RA).

3. Method according to claim 1 or 2,
- wherein the position of the boundary surface (FG) of the fan beam (F) is dynamically adjusted to the position of the boundary surface (NG) of the region (N) of the patient (13) to be imaged until the fan beam (F) is completely suppressed by the first diaphragm jaw (K1) and the second diaphragm jaw (K2) .

4. Method according to one of claims 1 to 3,
- wherein the movement (BK1) of the first diaphragm jaw (K1) takes place at a speed of at least 150 millimetres per second.

5. Method according to one of claims 1 to 4,
- wherein a movement (BK2) of the second diaphragm jaw (K2) during the capture (ED) of the projection data takes place in such a way that the position (FG1, FG2) of the boundary surface of the fan beam (F) can be dynamically adjusted to the position (NG1, NG2) of the boundary surface of the region (N) of the patient (13) to be imaged, such that the fan beam (F) penetrating the patient (13) outside the region to be imaged (N) is essentially avoided,
- wherein the positioning (PK2) of the second diaphragm jaw (K2) takes place in that a first rotational movement of a second threaded spindle (S2) is converted into a first translational movement of the second diaphragm jaw (K2) by means of a second threaded drive (T2), and wherein the movement (BK2) of the second diaphragm jaw (K2) takes place in that a second rotational movement of the second threaded spindle (S2) is converted into a second translational movement of the second diaphragm jaw (K2) by means of the second threaded drive (T2).

6. Method according to claim 5,
- wherein the first rotational movement of the second threaded spindle (S2) and the second rotational movement of the second threaded spindle (S2) are driven by means of a second stepper motor (M2),
- wherein the second rotational movement of the second threaded spindle (S1) is controlled in a field-oriented manner.

7. Method according to claim 5 or 6,
- wherein the movement (BK2) of the second diaphragm jaw (K1) takes place at a speed of at least 150 millimetres per second.

8. Computed tomography device (1), comprising:
- A radiation source (26) for generating radiation (27),
- A detector (28) and
- A collimator (K) with a first diaphragm jaw (K1) and a second diaphragm jaw (K2),
- wherein the collimator (K) is designed to position (PK1) the first diaphragm jaw (K1) and to position (PK2) the second diaphragm jaw (K2) in such a way that a layer collimation of a fan beam (F) of the radiation (27) can be set by means of the first diaphragm jaw (K1) and the second diaphragm jaw (K2),
- wherein the detector (28) is designed to capture (ED) the projection data of a region (N) of a patient (13) to be imaged, while the detector (28) and the radiation source (26) are rotated in a plane of rotation (RP) about an axis of rotation (RA) and at the same time the region (N) of the patient (13) to be imaged is moved relative to the plane of rotation (RP), wherein the fan beam (F) penetrates the region (N) of the patient (13) to be imaged and strikes the detector (28),
- wherein the collimator (K) is furthermore designed to move (BK1) the first diaphragm jaw (K1) during the capture (ED) of the projection data in such a way that a position (FG1, FG2) of a boundary surface of the fan beam (F) can be dynamically adjusted to a position (NG1, NG2) of a boundary surface of the region (N) of the patient (13) to be imaged, such that the fan beam (F) penetrating the patient (13) outside the region to be imaged (N) is essentially avoided,
- **characterised in that** the collimator (K) has a first threaded spindle (S1) and a first threaded drive (T1), wherein the first threaded drive (T1) is designed to convert a first rotational movement of the first threaded spindle (S1) into a first translational movement of the first diaphragm jaw (K1), by means of which the positioning (PK1) of the first diaphragm jaw (K1) takes place, and to convert a second rotational movement of the first threaded spindle (S1) into a second translational movement of the first diaphragm jaw (K1), by means of which translational movement the movement (BK1) of the first diaphragm jaw (K1) takes place,
- wherein the collimator (K) has a first stepper motor (M1) for driving the first rotational movement of the first threaded spindle (S1) and the second rotational movement of the first threaded spindle (S1),
- wherein the computed tomography device (1) further comprises a control unit (35), which is designed for field-oriented control of the second rotational movement of the first threaded spindle (S1).

9. Computed tomography device (1) according to claim 8,
- wherein the collimator (K) is designed, during the capture (ED) of the projection data, to keep the layer collimation of the fan beam (F) of the radiation (27) constant for at least one half revolution of the detector (28) and the radiation source (26) about the axis of rotation (RA).

10. Computed tomography device (1) according to claim 8 or 9,
- wherein the collimator (K) is designed to dynamically adapt the position of the boundary surface (FG) of the fan beam (F) to the position of the boundary surface (NG) of the region (N) of the patient (13) to be imaged until the fan beam (F) is completely suppressed by the first diaphragm jaw (K1) and the second diaphragm jaw (K2).

11. Computed tomography device (1) according to one of claims 8 to 10,
- wherein der collimator (K) is designed to move the first diaphragm jaw (K1) and/or the second diaphragm jaw (K2) in each case at a speed of at least 150 millimetres per second.

12. Computed tomography device (1) according to one of claims 8 to 11,
- wherein the collimator (K) is further designed to move (BK2) the second diaphragm jaw (K2) during the capturing (ED) of the projection data in such a way that the position (FG1, FG2) of the boundary surface of the fan beam (F) is dynamically adjusted to the position (NG1, NG2) of the boundary surface of the region (N) of the patient (13) to be imaged, such that the fan beam (F) penetrating the patient (13) outside the region to be imaged (N) is essentially avoided,
- wherein the collimator (K) has a second threaded spindle (S2) and a second threaded drive (T2), wherein the second threaded drive (T1) is designed to convert a first rotational movement of the second threaded spindle (S1) into a first translational movement of the second diaphragm jaw (K2), by means of which the positioning (PK2) of the second diaphragm jaw (K2) takes place, and to convert a second rotational movement of the second threaded spindle (S2) into a second translational movement of the second diaphragm jaw (K2), by means of which translational movement the movement (BK2) of the second diaphragm jaw (K2) takes place.

13. Computed tomography device (1) according to claim 12,
- wherein a thread pitch of the first threaded spindle (S1) is at least 3 millimetres per revolution.

14. Computed tomography device (1) according to claim 12 or 13,
- wherein the collimator (K) has a second stepper motor (M2) for driving the first rotational movement of the second threaded spindle (S2) and the second rotational movement of the second threaded spindle (S2),
- wherein the control unit (35) is designed for field-oriented control of the second rotational movement of the second threaded spindle (S2).

15. Computed tomography device (1) according to one of the claims 8 to 14,
- wherein a thread pitch of the first threaded spindle (S1) is at least 3 millimetres per revolution.

## Revendications

1. Procédé de détection de données de projection au moyen d'un appareil (1) de tomodensitométrie assisté par ordinateur, qui a une source (26) de rayonnement pour la production d'un rayonnement (27), un détecteur (28) et un collimateur (K) ayant une première mâchoire (K1) de diaphragme et une deuxième mâchoire (K2) de diaphragme, procédé dans lequel :
- on met (PK1) en position, la première mâchoire (K1) de diaphragme et on met (PK2) en position, la deuxième mâchoire (K2) de diaphragme de manière à régler, au moyen de la première mâchoire (K1) de diaphragme et de la deuxième mâchoire (K2) de diaphragme, une collimation de couche d'un faisceau (F) en éventail du rayonnement (27),
- on détecte (ED), au moyen du détecteur (28), les données de projection d'une partie (N) à représenter d'un patient (13) tandis que l'on fait tourner le détecteur (28) et la source (26) de rayonnement dans un plan (RP) de rotation autour d'un axe (RA) de rotation et que l'on déplacement en même temps la partie (N) à représenter du patient (13) par rapport au plan (RP) de rotation, dans lequel le faisceau (F) en éventail traverse la partie (N) à reproduire du patient (13) et arrive sur le détecteur (28),
- on déplace (BK1) la première mâchoire (K1) de diaphragme pendant la détection (ED) des données de projection de manière à adapter dynamiquement une position (FG1, FG2) d'une surface de démarcation du faisceau (F) en éventail à une position (NG1, NG2) d'une surface de démarcation de la partie (N) à représenter du patient (13) de façon à empêcher sensiblement que le faisceau (F) en éventail traverse le patient (13) à l'extérieur de la partie (N) à représenter,
- **caractérisé en ce que** le mise (PK1) en position de la première mâchoire (K1) de diaphragme s'effectue en transformant un premier mouvement de rotation d'une première broche (S1) filetée en un premier mouvement de translation de la première mâchoire (K1) de diaphragme au moyen d'un premier entraînement (T1) fileté et **en ce que** le mouvement (BK1) de la première mâchoire (K1) de diaphragme s'effectue en transformant un deuxième mouvement de rotation de la première broche (S1) filetée en un deuxième mouvement de translation de la première mâchoire (K1) de diaphragme au moyen du premier entraînement (T1) fileté,
- dans lequel on donne le premier mouvement de rotation de la première broche (S1) filetée et le deuxième mouvement de rotation de la première broche (S1) filetée au moyen d'un premier moteur (M1) pas à pas,
- dans lequel on règle le deuxième mouvement de rotation de la première broche (S1) filetée en fonction du champ.

2. Procédé suivant la revendication 1,
- dans lequel, lors de la détection (ED) des données de projection, on maintient constante la collimation de couche du faisceau (F) en éventail du rayonnement (27) pendant au moins un demi-tour du détecteur (28) et de la source (26) de rayonnement autour de l'axe (RA) de rotation.

3. Procédé suivant la revendication 1 ou 2,
- dans lequel on adapte la position de la surface (FG) de démarcation du faisceau (F) en éventail dynamiquement à la position de la surface (NG) de démarcation de la partie (N) à représenter du patient (13), jusqu'à un diaphragme complet du faisceau (F) en éventail par la première mâchoire (K1) de diaphragme et par la deuxième mâchoire (K2) de diaphragme.

4. Procédé suivant la revendication 1 à 3,
- dans lequel le déplacement (BK1) de la première mâchoire (K1) de diaphragme s'effectue à une vitesse d'au moins 150 millimètres à la seconde.

5. Procédé suivant la revendication 1 à 4,
- dans lequel un déplacement (BK2) de la deuxième mâchoire (K2) de diaphragme, pendant la détection (ED) des données de protection, s'effectue de manière à adapter dynamiquement la position (FG1, FG2) de la surface de démarcation du faisceau (F) en éventail à la position (NG1, NG2) de la surface de démarcation de la partie (N) à représenter du patient (13) de façon à empêcher sensiblement que le faisceau (F) en éventail ne traverse le patient (13) à l'extérieur de la partie (N) à représenter,
- dans lequel la mise (PK2) en position de la deuxième mâchoire (K2) de diaphragme s'effectue en transformant un premier mouvement de rotation d'une deuxième broche (S2) filetée en un premier mouvement de translation de la deuxième mâchoire (K2) de diaphragme au moyen d'un deuxième entraînement (T2) fileté et dans lequel le déplacement (BK2) de la deuxième mâchoire (K2) de diaphragme s'effectue en transformant un deuxième mouvement de rotation de la deuxième broche (S2) filetée en un deuxième mouvement de translation de la deuxième mâchoire (K2) de diaphragme au moyen du deuxième entraînement (T2) fileté.

6. Procédé suivant la revendication 5,
- dans lequel on donne le premier mouvement de rotation de la deuxième broche (S2) filetée et le deuxième mouvement de rotation de la deuxième broche (S2) filetée au moyen d'un deuxième moteur (M2) pas à pas,
- dans lequel on règle le deuxième mouvement de rotation de la deuxième broche (S2) filetée en fonction du champ.

7. Procédé suivant la revendication 5 ou 6,
- dans lequel le mouvement (BK2) de la deuxième mâchoire (K2) de diaphragme s'effectue à une vitesse d'au moins 150 millimètres à la seconde.

8. Appareil (1) de tomodensitométrie assisté par ordinateur, comportant :
- une source (26) de rayonnement pour la production d'un rayonnement (27),
- un détecteur (28) et
- un collimateur (K) ayant une première mâchoire (K1) de diaphragme et une deuxième mâchoire (K2) de diaphragme,
- dans lequel le collimateur (K) est constitué pour mettre (PK1) en position la première mâchoire (K1) de diaphragme et pour mettre (PK2) en position la deuxième mâchoire (K2) de diaphragme de manière à pouvoir régler une collimation de couche d'un faisceau (F) en éventail du rayonnement (27) au moyen de la première mâchoire (K1) de diaphragme et de la deuxième mâchoire (K2) de diaphragme,
- dans lequel le détecteur (28) est constitué pour la détection (ED) des données de projection d'une partie (N) à représenter d'un patient (13) tandis que l'on fait tourner le détecteur (28) et la source (26) de rayonnement dans un plan (RP) de rotation autour d'un axe (RA) de rotation et que l'on déplace en même temps la partie (N) à représenter du patient (13) par rapport au plan (RP) de rotation, le faisceau (F) en éventail traversant la partie (N) à représenter du patient (13) et arrivant sur le détecteur (28),
- dans lequel le collimateur (K) est constitué en outre pour déplacer (BK1) la première mâchoire (K1) de diaphragme pendant la détection (ED) des données de projection de manière à pouvoir adapter dynamiquement une position (FG1, FG2) d'une surface de démarcation du faisceau (F) en éventail à une position (NG1, NG2) d'une surface de démarcation de la partie (N) à représenter du patient (13) de façon à empêcher sensiblement que le faisceau (F) en éventail ne traverse le patient (13) à l'extérieur de la partie (N) à représenter,
- **caractérisé en ce que** le collimateur (K) a une première broche (S1) filetée et un premier entraînement (T1) fileté, dans lequel le premier entraînement (T1) fileté est constitué pour transformer un premier mouvement de rotation de la première broche (S1) filetée en un premier mouvement de translation de la première mâchoire (K1) de diaphragme, par lequel la mise (PK1) en position de la première mâchoire (K1) de diaphragme a lieu, et pour transformer un deuxième mouvement de rotation de la première broche (S1) filetée en un deuxième mouvement de translation de la première mâchoire (K1) de diaphragme, par lequel le déplacement (BK1) de la première mâchoire (K1) de diaphragme a lieu,
- dans lequel le collimateur (K) a un premier moteur (M1) pas à pas pour donner le premier mouvement de rotation à la première broche (S1) filetée et le deuxième mouvement de rotation a la première broche (S1) filetée,
- dans lequel l'appareil (1) de tomodensitométrie assisté par ordinateur a en outre une unité (35) de régulation, qui est constituée pour la régulation en fonction du champ du deuxième mouvement de rotation de la première broche (S1) filetée.

9. Appareil (1) de tomodensitométrie assisté par ordinateur suivant la revendication 8,
- dans lequel le collimateur (K) est constitué pour maintenir constante, lors de la détection (ED) des données de projection, la collimation de couche du faisceau (F) en éventail du rayonnement (27) pendant au moins un demi-tour du détecteur (28) et de la source (26) de rayonnement autour de l'axe (RA) de rotation.

10. Appareil (1) de tomodensitométrie assisté par ordinateur suivant la revendication 8 ou 9,
- dans lequel le collimateur (K) est constitué pour adapter la position de la surface (FG) de démarcation du faisceau (F) en éventail dynamiquement à la position de la surface (NG) de démarcation de la partie (N) à représenter du patient (13), jusqu'à un diaphragme complet du faisceau (F) en éventail par la première mâchoire (K1) de diaphragme et la deuxième mâchoire (K2) de diaphragme.

11. Appareil (1) de tomodensitométrie assisté par ordinateur suivant l'une des revendications 8 à 10,
- dans lequel le collimateur (K) est constitué pour déplacer la première mâchoire (K1) de diaphragme et/ou la deuxième mâchoire (K2) de diaphragme respectivement à une vitesse d'au moins 150 millimètres à la seconde.

12. Appareil (1) de tomodensitométrie assisté par ordinateur suivant l'une des revendications 8 à 11,
- dans lequel le collimateur (K) est constitué en outre pour déplacer (BK2) la deuxième mâchoire (K2) de diaphragme pendant la détection (ED) des données de projection de manière à pouvoir adapter dynamiquement la position (FG1, FG2) de la surface de démarcation du faisceau (F) en éventail à la position (NG1, NG2) de la surface de démarcation de la partie (N) à représenter du patient (13), de façon à empêcher sensiblement que le faisceau (F) en éventail ne traverse le patient (13) à l'extérieur de la partie (N) à représenter,
- dans lequel le collimateur (K) a une deuxième broche (S2) filetée et un deuxième entraînement (T2) fileté, dans lequel le deuxième entraînement (T2) fileté est constitué pour transformer un premier mouvement de rotation de la deuxième broche (S2) filetée en un premier mouvement de translation de la deuxième mâchoire (K2) de diaphragme, par lequel la mise (PK2) en position de la deuxième mâchoire (K2) de diaphragme a lieu, et un deuxième mouvement de rotation de la deuxième broche (S2) filetée en un deuxième mouvement de translation de la deuxième mâchoire (K2) de diaphragme par lequel le mouvement (BK2) de la deuxième mâchoire (K2) de diaphragme a lieu.

13. Appareil (1) de tomodensitométrie assisté par ordinateur suivant la revendication 12,
- dans lequel un pas du filetage de la deuxième broche (S2) filetée est d'au moins 3 millimètres par tour.

14. Appareil (1) de tomodensitométrie assisté par ordinateur suivant la revendication 12 ou 13,
- dans lequel le collimateur (K) a un deuxième moteur (M2) pas à pas pour donner le premier mouvement de rotation de la deuxième (S2) filetée et le deuxième mouvement de rotation de la deuxième broche (S2) filetée,
- dans lequel l'unité (35) de régulation est constituée pour la régulation en fonction du champ du deuxième mouvement de rotation de la deuxième broche (S2) filetée.

15. Appareil (1) de tomodensitométrie assisté par ordinateur suivant l'une des revendications 8 à 14,
- dans lequel un pas du filet de la première broche (S1) filetée est d'au moins 3 millimètres par tour.
